Europäisches Patentamt

European Patent Office

Office européen des brevets

(19)

(11)  EP 0 763 726 A2

(12)  **EUROPÄISCHE PATENTANMELDUNG**

(43) Veröffentlichungstag:
     **19.03.1997  Patentblatt 1997/12**

(51) Int. Cl.⁶: **G01N 11/00**,  G01N 33/38

(21) Anmeldenummer: 96113900.3

(22) Anmeldetag: **30.08.1996**

(84) Benannte Vertragsstaaten:
     **AT BE DE DK FR GB**

(30) Priorität: **01.09.1995 DE 19532248**

(71) Anmelder: **Rheinische Kalksteinwerke GmbH**
     **42489 Wülfrath (DE)**

(72) Erfinder:
     • **Schwertmann, Thomas, Dipl.-Ing.**
       **42781 Haan (DE)**
     • **Koop, Wolfgang**
       **42489 Wülfrath (DE)**

(54)  **Vorrichtung zur Messung der Eigenschaften von fliessfähigen Schüttgütern**

(57)  Bei bekannten Vorrichtungen zur Messung der Eigenschaften von Schüttgütern nach dem Prinzip des einachsigen vertikalen Druckversuchs sind die Messungen kosten- und zeitaufwendig. Zur Bedienung wird qualifiziertes Personal benötigt. Die Geräte sind daher für laufende Kontrollen weniger geeignet. Die neue Vorrichtung soll so gestaltet sein, daß auf einfache Weise eine definiert verdichtete zylindrische Schüttgutprobe ermöglicht wird und die Fließfähigkeiten von Schüttgütern unterschiedlicher Körnungen direkt miteinander vergleichbar werden.

Die hohlzylindrische Form, in der die Schüttgutprobe verdichtet wird, ist in Belastungsrichtung reibungsarm verschiebbar und in jeder Position durch ein Gegengewicht genau kompensierbar.

Die Vorrichtung eignet sich besonders zur Durchführung von Messungen im Rahmen der Qualitätsüberwachung.

Fig. 1

**Beschreibung**

Die Erfindung betrifft eine Vorrichtung zur Messung der Eigenschaften von Schüttgütern nach dem Prinzip des einachsigen vertikalen Druckversuchs sowie Verfahren zur Messung der Fließfähigkeit, der Schüttdichte und des Zeitverfestigungsverhaltens von Schüttgut mit dieser Vorrichtung.

Die Fließfähigkeit, insbesondere von technisch trockenen, pulverförmigen Schüttgütern ist eine wichtige Materialeigenschaft. Beim erreichten Stand der Prozeßautomatisierung ist die Kenntnis der Eigenschaften von Schüttgütern notwendig für eine sichere Auslegung von Silos, Zwischenbehältern, Förderorganen usw. insbesondere unter dem Aspekt der Funktionssicherheit ohne manuellen Eingriff.

Darüber hinaus ist die Fließfähigkeit eines Schüttgutes oft eine wichtige anwendungstechnische Eigenschaft. Eine anwendungstechnische Eigenschaft ist u. a. durch ihre reproduzierbare Meßbarkeit charakterisiert, dieser Aspekt gewinnt durch zunehmende Installation von Qualitätssicherungssystemen nach DIN ISO 9000 bis 9002 an Bedeutung. In vielen Fällen wird die Fließfähigkeit eines Schüttgutes gezielt beeinflußt, z. B. über die Korngrößenverteilung, Zusatz von Fließhilfsmitteln, die Produktfeuchte usw. Dies erfordert zwingend eine Messung der Zielgröße "Fließfähigkeit" vor allem im Rahmen der Qualitätsüberwachung, aber auch bei Entwicklungsarbeiten.

Die reproduzierbare Messung der Fließfähigkeit ist jedoch, z. B. im Vergleich zur Messung der Viskosität von Flüssigkeiten, schwierig, da ein Schüttgut allgemein ein 3-Phasen-System ist (disperser Feststoff, Gas, Oberflächenfeuchte). Die feste, disperse Phase ist dabei meist durch eine schwer beschreibbare Formenvielfalt gekennzeichnet.

Die Fließfähigkeit als eine summarische Schüttguteigenschaft ist daher durch eine Vielzahl von Einflußgrößen bestimmt. Oft kann dasselbe pulverförmige Schüttgut in Abhängigkeit vom Verdichtungszustand einen fluidähnlichen bis festkörperähnlichen Zustand annehmen.

Für die vielfältigen praktischen Anwendungen sind mehrere Methoden zur Messung der Fließfähigkeit von Schüttgütern bekannt, die zum Teil auf unterschiedlichen Meßprinzipien beruhen und verschiedene physikalische Größen erfassen.

In der Kalk- und Zementindustrie verbreitet ist die Prüfung des Fließverhaltens nach IMSE. Das Prüfverfahren dient der innerbetrieblichen Ermittlung der Fließfähigkeit feinteiliger Kalkprodukte. Es liefert keine zwischen mehreren Prüfstellen vergleichbare Absolutwerte. Vorteil dieser Methode ist der geringe Zeitbedarf einer Messung und der einfache apparative Aufbau (niedrige Kosten).

Der Zeitbedarf der IMSE-Methode gemäß gültigem Arbeitsblatt des Bundesverbandes der deutschen Kalkindustrie - dort ist Mittelwertbildung aus 3 Einzelbestimmungen vorgeschrieben - liegt bei etwa 15 min. (Einzelmessung ca. 5 min.)

In der Praxis zeigen sich jedoch folgende Probleme:

- Bearbeiterabhängigkeit der Meßergebnisse.
- Standortabhängigkeit der Meßergebnisse (dynamisches Verfahren).
- Fließfähigkeiten nach IMSE für Schüttgüter unterschiedlicher Feinheit können nicht direkt verglichen werden.
- Die Fließfähigkeit wird in undefiniert aufgelockertem Zustand bestimmt. In der Praxis ist aber oft die Fließfähigkeit in entlüftetem, verdichtetem Zustand (z. B. bei Silolagerung) gefragt. Hier ist die Fließfähigkeit nach IMSE oft wenig aussagekräftig.

Eine genauere, in Bezug auf viele Fragestellungen quantitativ aussagekräftigere Messung der Fließfähigkeit von Schüttgütern ist mit sogenannten Schergeräten möglich. Ein bekanntes Gerät ist z. B. das Translationsschergerät nach JENIKE.

Allerdings werden diese Schergeräte für Zwecke der Qualitätskontrolle wegen des hohen Zeitbedarfs für die einzelne Messung bzw. der hohen Kosten insbesondere für automatisierte Geräte selten eingesetzt.

Die Herstellung einer für Meßzwecke geeigneten Schüttgutprobe in einer zylindrischen Form im einachsigen Druckversuch ist insbesondere bei feinkörnigen Schüttgütern wegen der Reibung zwischen Schüttgut und Formoberfläche nicht unproblematisch. Wird eine auf einer ebenen Fläche aufstehende zylindrische Form mit Schüttgut gefüllt und anschließend die Schüttgutoberfläche mit einer definierten Verfestigungsspannung $\sigma_1$ (Normalspannung) belastet, so nimmt die in Richtung der Formachse wirksame Normalspannung mit zunehmendem Abstand von der Probenoberfläche ab, da zwischen Schüttgut und Formoberfläche Reibungskräfte wirksam werden. Die Festigkeit einer Schüttgutprobe ist jedoch durch die wirksame Verfestigungsspannung $\sigma_1$ bestimmt, dieser Zusammenhang ist als Gut-Festigkeits-Kennlinie oder Fließfunktion aus dem JENIKE-Verfahren zur Auslegung von Silos bekannt. Insofern wäre für Meßzwecke eine über die Höhe der zylindrischen Schüttgutprobe konstante Verfestigungsspannung ideal.

Bei der aus WO 94/08220 A1 bekannten Vorrichtung wird zur Verringerung der Reibung zwischen Schüttgutprobe und Formwand eine elastische Membran vorgesehen. Zwischen der Membran und der Formwand kann sich als Schmiermittel z. B. ein dünner Ölfilm befinden. Es finden sich aber keine Angaben über die Größe der noch wirksamen Wandschubspannungen. Der für eine Messung erforderliche Zeitaufwand beträgt ca. 20 min, ein in der industriellen Praxis noch einigermaßen akzeptabler Wert. Allerdings erscheint die Apparatur für eine industrielle, vor allem produk-

EP 0 763 726 A2

tionsnahe Qualitätskontrolle noch von den Kosten her zu aufwendig und in der Bedienung zu schwierig, da qualifiziertes Personal erforderlich ist.

Zur Überwindung der Nachteile bei Vorrichtungen nach Stand der Technik wird daher eine möglichst einfache Methode zur Herstellung einer definiert verdichteten zylindrischen Schüttgutprobe angestrebt. Die Vorrichtung sollte sich auszeichnen durch einfachen, robusten Aufbau, gute Reproduzierbarkeit der Meßergebnisse, kurze Meßzeiten und einfache Handhabung.

Die Aufgabe der Erfindung besteht insbesondere darin, Fließfähigkeiten von Schüttgütern unterschiedlicher Körnungen direkt miteinander vergleichbar zu machen und durch Vergleich mit der mittels Schergerät ermittelten Fließfähigkeit eine Kalibrierungsmöglichkeit zu schaffen.

Die Aufgabe wird dadurch gelöst, daß bei der erfindungsgemäßen Vorrichtung zur Messung der Eigenschaften von Schüttgütern die hohlzylindrische Form (1) gegenüber dem feststehenden Unterstempel (3) während des Verdichtungsvorganges in Belastungsrichtung verschiebbar ist. Die zylindrische Schüttgutprobe wird dabei auf einfache Weise von beiden Stirnseiten her gleichmäßig belastet. Daraus resultiert gegenüber der Belastung der Schüttgutprobe nur von einer Stirnseite her eine Vergleichmäßigung der Normalspannung $\sigma_1$ über die Probenhöhe. Damit wird eine für die Praxis ausreichende Annäherung an den Idealfall einer über die Probenhöhe konstanten Normalspannung $\sigma_1$ erreicht.

Bei einer bevorzugten Ausführungsform ist die Gewichtskraft der in Belastungsrichtung gegenüber dem Unterstempel (3) verschiebbaren Form (1) in jeder Position durch eine gleich große Gegenkraft genau kompensierbar. Die Gewichtskompensation erfolgt also nicht durch Federkraft sondern wegunabhängig z. B. durch ein über einen Hebel wirkendes Gegengewicht. Dabei entspricht in jeder Stellung der Form die Gewichtskraft der Form genau der Gewichtskraft des Gegengewichts.

Durch diese Gewichtskompensation wird erreicht, daß über die Form keine Zusatzbeanspruchung der Schüttgutprobe durch Wandschubspannungen erfolgt. Die Schüttgutprobe wird lediglich mit der bekannten und wählbaren Normalspannung $\sigma_1$ belastet, die Summe der zwischen Form und Schüttgutoberfläche wirksamen vertikalen Wandschubspannungen ist nach Abschluß des Verdichtungsvorgangs gleich Null.

Die Form kann mehrteilig, vorzugsweise dreiteilig sein, wobei die Teilfugen parallel zur Zylinderachse verlaufen und in einem Schnitt senkrecht zur Zylinderachse mit Radialstrahlen zusammenfallen.

Als weitere Ausgestaltung kann die Innenfläche der Form mit einer Edelstahlfolie ausgekleidet sein. Eine besonders glatte und harte Oberfläche ist vorteilhaft zur Erzielung eines niedrigen Gut-Wand-Reibungswinkels.

Damit die während der Verdichtung von der Schüttgutprobe abgepreßte Luft über die Stirnseiten der Schüttgutprobe abströmen kann, ist es zweckmäßig, daß die jeweils zwischen Unterstempel und Verdichtungsdeckel angeordneten stirnseitigen Begrenzungsplatten luftdurchlässig sind. Sie dürfen sich aber unter den mit der erfindungsgemäßen Vorrichtung erreichbaren Drücken nicht verformen. Der Verdichtungsdeckel sollte vorzugsweise aus einer Aluminiumlegierung gefertigt sein.

Als weitere Ausgestaltung kann zwischen den luftdurchlässigen Begrenzungsplatten und den Stirnseiten der Schüttgutprobe ein leicht auswechselbares Filter angeordnet sein, vorzugsweise aus Filtergewebe, Filtervlies oder Filterpapier.

Ein Verfahren zur Messung der Fließfähigkeit von Schüttgut mit der erfindungsgemäßen Vorrichtung kann dadurch gekennzeichnet sein, daß nach Entfernen der Form eine langsam und kontinuierlich steigende Belastung der Schüttgutprobe bis zum Bruch erfolgt, wobei die Belastung als gleichmäßig verteilte Druckspannung auf die obere Stirnseite der Schüttgutprobe wirkt und die im Moment des Bruches wirkende Druckspannung $f_c$ gemessen wird.

Mit der erfindungsgemäßen Apparatur wird die einaxiale Druckfestigkeit $f_c$ einer zuvor unter der Druckspannung $\sigma_1$ definiert verdichteten zylindrischen Schüttgutprobe gemessen, d. h., es werden die aus der Schüttgutmechanik bekannten, physikalisch begründeten, Größen $f_c$ und $\sigma_1$ gemessen.

Daher besteht durch Vergleichsmessungen mit der erfindungsgemäßen Vorrichtung und dem JENIKE-Schergerät eine Kalibriermöglichkeit. Gleiche Ergebnisse sind im allgemeinen nicht zu erwarten, da sich die Verfestigungsprozeduren unterscheiden. Für den Einsatz zu Zwecken der Qualitätsüberwachung (z. B. Kontrolle der Fließhilfsmitteldosierung) ist eine Kalibrierung jedoch nicht notwendig.

Es hat sich herausgestellt, daß die Differenzierungsfähigkeit der erfindungsgemäßen Apparatur wesentlich höher ist, als beim Verfahren nach IMSE. Physikalisch zu erwartende Abhängigkeiten, z. B. Fließfähigkeiten als Funktion der Feinheit bei im übrigen gleichen Parametern werden richtig wiedergegeben.

Hervorzuheben ist die gute Reproduzierbarkeit. Der Zeitbedarf liegt bei etwa 12 min. für 1 Messung. Wegen der guten Reproduzierbarkeit ist nur 1 Messung erforderlich.

Die erfindungsgemäße Vorrichtung dient nicht nur zur Messung der Fließeigenschaften, es kann auch die Schüttdichte als Funktion der Verfestigungsspannung durch Ermittlung des Volumens der verdichteten Probe (2*) und Ermittlung der Masse der Probe bestimmt werden. Außerdem kann das Zeitverfestigungsverhalten mit der erfindungsgemäßen Vorrichtung bestimmt werden, indem die Druckfestigkeit der verdichteten Schüttgutprobe (2*) nach Beaufschlagung mit der Verdichtungslast (4) für eine wählbare Zeitdauer bestimmt wird.

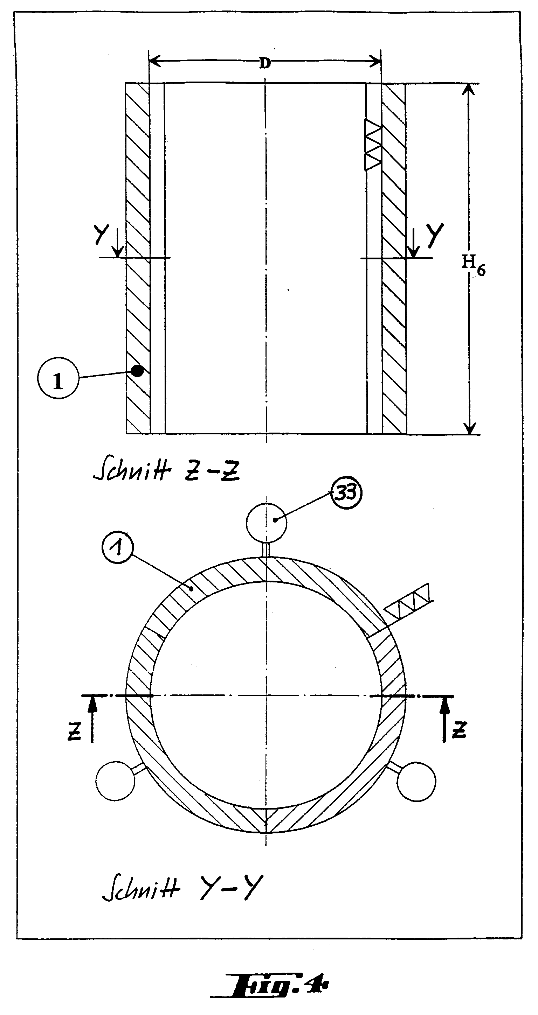

Die Erfindung wird anhand der Zeichnungen Fig. 1 bis Fig. 5 näher erläutert.

Fig. 1 zeigt Längsschnitt und Seitenansicht der erfindungsgemäßen Meßapparatur.

Fig. 2 zeigt den Verdichtungsdeckel (4.1).

Fig. 3 zeigt die Vorrichtung zur Aufbringung der Bruchlast.

Fig. 4 zeigt die Form (1) im Längsschnitt und in der Draufsicht.

Fig. 5 zeigt Einzelheiten zur Versuchsdurchführung (5.1 bis 5.3).

In einem dickwandigen Rohrstück (30) ist mittig ein Haltering (31) angeordnet, der über eine Hohlwelle (10) den Unterstempel (3) trägt. Der Unterstempel (3), auf dessem hochgezogenen Rand die untere Begrenzungsplatte (15) aufliegt, trägt die Schüttgutprobe (2) bzw. (2*). Schüttgutprobe (2*) ist die Schüttgutprobe (2) nach Arretieren von Teller (5), entfernen von Füllring (8), Abstreichen des überstehenden Teils der Schüttgutprobe (2), lösen von Spannring (7) und Entfernen von Form (1).

An Rohrstück (30) sind 3 Stellfüße (25) angeordnet, die eine exakt horizontale Ausrichtung der Oberfläche von Unterstempel (3) erlauben.

Der feststehende Unterstempel (3) wird von einer beispielsweise dreiteiligen zylindrischen Form (1) umschlossen. Die Form (1) steht auf Teller (5) auf. Teller (5) und damit Form (1) sind in Richtung der vertikalen Formachse beweglich angeordnet. Dies wird durch ein handelsübliches Linearkugellager (9) erreicht. Die Außenfläche der Hohlwelle (10) bildet die Kugellauffläche. Die Bohrung im Teller (5), die das Linearkugellager (9) aufnimmt, wurde so toleriert, daß sich einerseits ein möglichst leichter Lauf, andererseits aber kein Radialspiel einstellte. Idealerweise erfolgt die axiale Bewegung von Form (1) bzw. Teller (5) reibungsfrei und ohne jegliches Radialspiel. Diese Zielsetzung muß bei Auswahl der Linearführung beachtet werden.

Die Gewichtskraft von Form (1) und aufgesetztem Füllring (8) wird durch eine gleich große, entgegengerichtete, wegunabhängig wirkende Gegenkraft gerade ausgeglichen. Konstruktiv kann die Kompensation der Gewichtskraft der Form (1) beispielsweise dadurch erreicht werden, daß Teller (5) an zwei Punkten durch handelsübliche Kugellager (11) unterstützt wird. Diese Kugellager sind an einem Wägebalken (12) montiert, dessen Drehpunkt A wie bei einer Balkenwaage als Schneide/Pfanne ausgebildet ist. Bei einem Hebelarmverhältnis von z. B. 1:1 muß dann Kontergewicht (13) dem Gesamtgewicht von Form (1), Teller (5), Zentrierring (6) und Linearkugellager (9) entsprechen. Mittelpunkt von Kugellager (11), Drehpunkt A und Angriffspunkt B der Gewichtskraft von Kontergewicht (13) liegen dabei auf einer Geraden.

Form (1) wird auf Teller (5) durch den (abnehmbaren) Zentrierring (6) fixiert, dessen Außendurchmesser dem Innendurchmesser der Form (1) entspricht. Während des Verdichtungsvorgangs wird Form (1) durch einen leicht und erschütterungsfrei demontierbaren Spannring (7) zusammengehalten.

Die Form (1) muß exakt gefertigt sein, insbesondere müssen die Stirnflächen planparallel zueinander sein, der Innendurchmesser über die Höhe gleichbleibend sein und die Teilfugen präzise bearbeitet sein. Nach Zusammenschieben der Formteile auf Teller (5) (Positionierung durch Zentrierring (6)) dürfen sich diese beim Anziehen von Spannring (7) nicht bewegen. Die Innenfläche der Form (1) ist idealerweise glatt und hart, was z. B. durch Oberflächenhärtung und anschließendes Feinschleifen erreicht werden kann. Zur Erzielung einer kleinen Rauhtiefe können die bekannten Verfahren des Schleifens, Honens und Feinschleifens (Läppen) eingesetzt werden. Die Form (1) der erfindungsgemäßen Vorrichtung kann aus Normalstahl gefertigt sein und beispielsweise einen Innendurchmesser D = 95 mm aufweisen. Selbstverständlich kann auch ein anderer Formdurchmesser verwendet werden, da für das Meßprinzip lediglich das Verhältnis H/D maßgeblich ist. Die Wahl von Oberflächenqualität und -material muß so erfolgen, daß die Haftkräfte zwischen Formoberfläche und Schüttgut möglichst klein sind, so daß die Schüttgutprobe (2*) nach Abschluß des Verdichtungsvorgangs beschädigungsfrei ausgeschalt werden kann. Dies kann erfindungsgemäß dadurch erreicht werden, daß die Innenfläche der Form (1) mit einer Edelstahlfolie ausgekleidet ist.

Für die untersuchten Schüttgüter - Kalkhydrate, Kalksteinmehle und Weißfeinkalk - erwies sich die Edelstahlfolie als sehr gut geeignet. Versuche mit verschiedenen sehr glatten Kunststoffolien - insbesondere einer selbstklebenden Kunststoffolie mit Teflon-Oberfläche waren weniger erfolgreich.

Der Außendurchmesser des Unterstempels (3) ist etwa 0,2 mm kleiner als der Innendurchmesser von Form (1) gewählt, so daß Form (1) bei ihrer Bewegung während des Verdichtungsvorgangs den Unterstempel nicht berührt.

Funktionswichtig ist die luftdurchlässige Ausführung der stirnseitigen Begrenzungsplatten (15, 16) von Schüttgutprobe (2). Die während des Verdichtungsvorgangs aus der Schüttgutprobe (2) abgepreßte Luft kann so großflächig abströmen. Vorversuche mit luftundurchlässigen stirnseitigen Begrenzungsplatten hatten gezeigt, daß dann insbesondere bei feinteiligen Schüttgütern niedriger Schüttdichte (Schüttung enthält viel Luft) die über die Ringspalte zwischen Form (1) und Unterstempel (3) bzw. Verdichtungsdeckel (4.1) austretende Luft Schüttgut mitreißt, da wegen der kleinen Fläche der Ringspalte kurzzeitig hohe Strömungsgeschwindigkeiten auftreten.

Die Luftdurchlässigkeit der stirnseitigen Begrenzungsplatten (15, 16) der Schüttgutprobe kann vorzugsweise dadurch erreicht werden, daß diese als Lochbleche ausgebildet werden. Bei einer bevorzugten Ausführungsform übersteigt die Gesamtquerschnittsfläche der Bohrungen R im Verdichtungsdeckel (4.1) die Gesamtquerschnittsfläche der Bohrungen in der oberen Begrenzungsplatte (16). Möglich ist aber auch beispielsweise die Verwendung von Sintermetallplatten. Zwischen den durchbrochenen Begrenzungsplatten (15, 16) und den Stirnseiten der Schüttgutprobe können Filter hoher Luftdurchlässigkeit (17) angeordnet sein. Diese Filter werden vor jedem Versuch erneuert. Die abgepreßte Luft kann durch Kanal K des Unterstempels (3) bzw. Bohrungen R des Verdichtungsdeckels (4.1) abströmen.

Für die Begrenzungsplatten der Schüttgutprobe können Lochbleche nach DIN 3310/2 (Loch $\varnothing$ 2,0 mm, freier Querschnitt $a_0$ = 35 %) verwendet werden. Auf die Form (1), die durch den leicht demontierbaren Spannring (7) zusammengehalten wird, wird Füllring (8) aufgesetzt. Der Innendurchmesser $D_1$ des Füllrings (8) ist etwa 0,2 mm kleiner als der Innendurchmesser der Form (1). Dadurch ist eine Behinderung der Relativbewegung zwischen Schüttgut und Form (1) während der Komprimierung durch eine vorstehende Kante ausgeschlossen.

Die Höhe $H_1$ des Füllrings (8) ist so gewählt, daß nach Abschluß des Verdichtungsvorgangs von Schüttgutprobe (2) deren Oberkante an jeder Stelle wenigstens 2 mm, aber höchstens 8 mm oberhalb der Oberkante von Form (1) liegt. Daraus ergibt sich eine Materialabhängigkeit der Höhe $H_1$ des Füllrings (8). Zur Vorrichtung gehört daher ein Satz gewichtsgleicher Füllringe, deren Höhe beginnend von $H_1$=5 mm z. B. in 5 mm-Schritten gestaffelt ist.

Die Verfestigungsspannung $\sigma_1$ wird über einen Verdichtungsdeckel (4.1), der mittig durch die Verdichtungslast (4) belastet wird, auf die Oberfläche der Schüttgutprobe (2) aufgebracht.

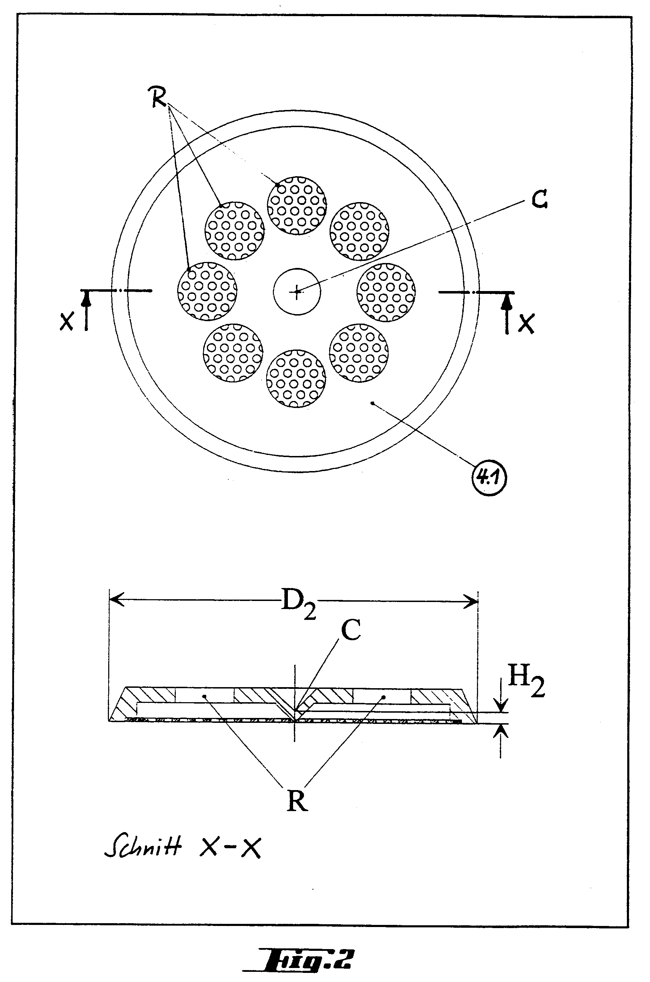

Vorteilhaft ist, wenn der Abstand des Kraftangriffspunktes C der Verdichtungslast (4) zur Kontaktfläche zwischen Schüttgutoberfläche und Unterseite des Verdichtungsdeckels (4.1) möglichst klein ist. Charakteristischerweise ist bei dem in Fig. 2 gezeigten Verdichtungsdeckel (4.1) dieser Abstand $H_2 \leq 3$ mm. Der Verdichtungsdeckel kann aus einer Aluminiumlegierung gefertigt sein.

Die wirksame Verfestigungsspannung $\sigma_1$ errechnet sich aus der Gesamtgewichtskraft von Verdichtungsdeckel (4.1), Verdichtungslast (4) (incl. Bolzen (4.2) und Kappe (4.3)) und Zusatzgewicht(en) (4.4) dividiert durch die Kreisfläche mit Durchmesser D. Die Verfestigungsspannung $\sigma_1$ kann über die Zusatzgewichte (4.4), deren Staffelung der jeweiligen Aufgabenstellung angepaßt werden kann, einfach variiert werden.

Die Masse der Verdichtungslast (4) kann so gewählt werden, daß sich ein zweckmäßiger Minimalwert der Verdichtungsspannung $\sigma_1$ ergibt. Dieser Minimalwert wird vorzugsweise so gewählt, daß er den mit einem Translationsschwergerät nach JENIKE erreichbaren Minimalwerten von etwa 3000 - 4000 Pa entspricht. In Sonderfällen kann es interessant sein, den Minimalwert durch geeignete Konstruktion und Werkstoffwahl deutlich niedriger zu wählen, was ohne großen Aufwand möglich ist.

Soll z. B. die kleinste Verfestigungsspannung $\sigma_{1, min}$ = 3000 Pa betragen, so muß bei einem Durchmesser D = 95 mm die Gesamtmasse von Verdichtungsdeckel (4.1) und Verdichtungslast (4) (incl. Bolzen (4.2) und Kappe (4.3)) 2167,6 g betragen. Soll $\sigma_1$ in Schritten von z. B. 500 Pa variiert werden können, so beträgt die Masse eines Zusatzgewichtes (4.4) 361,3 g.

Der Schwerpunkt $S_V$ der Verdichtungslast (4) liegt vorzugsweise wenigstens 10 mm unterhalb des Kraftangriffspunktes C ($H_3 > 10$ mm).

Die Verdichtungslast (4) der erfindungsgemäßen Apparatur kann glockenförmig ausgebildet und als (rotationssymmetrisches) Drehteil hergestellt sein. Dadurch ist die Zentrierung der Durchgangsbohrung für Bolzen (4.2) in Verdichtungslast (4) sowie der Bohrung für die Schnur (32) in Kappe (4.3) unproblematisch. Die so hergestellte Verdichtungslast richtet sich nach Aufsetzen auf Verdichtungsdeckel (4.1) automatisch so aus, daß die Achse von Bolzen (4.2) lotrecht ist, d. h. die Gewichtskraft wird mittig und lotrecht auf den Verdichtungsdeckel aufgebracht.

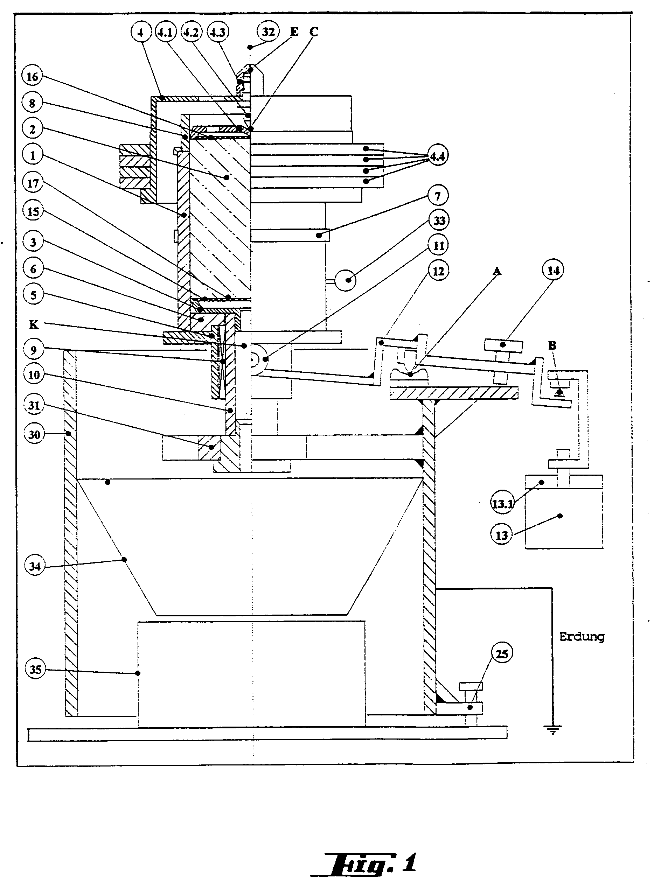

Bei einer Ausführungsform der erfindungsgemäßen Apparatur wurde die Verdichtungslast (4) aus Normalstahl gefertigt mit einer Masse von 2827 g (incl. Bolzen (4.2) und Kappe (4.3)). Daraus ergibt sich mit Formdurchmesser D = 95 mm eine minimale Verdichtungsspannung $\sigma_{1, min}$ = 3912 N/m$^2$. Bei gleichen Abmessungen, jedoch unter Verwendung des Werkstoffs Aluminium ergibt sich ein etwa um den Faktor 2,9 kleinerer Wert, d. h. $\sigma_{1, min} \sim 1350$ N/m$^2$. Eine derart kleine Verfestigungsspannung kann manchmal von Interesse sein. Grundsätzlich kann die Verdichtungslast (4) konstruktiv auch anders als nach Fig. 1 ausgebildet sein (z. B. Hängevorrichtung des Translationsschergerätes nach JENIKE). Wichtig ist nur, daß der Schwerpunkt der Verdichtungslast (4) deutlich unterhalb des Kraftangriffspunktes C liegt ($H_3 > 10$ mm).

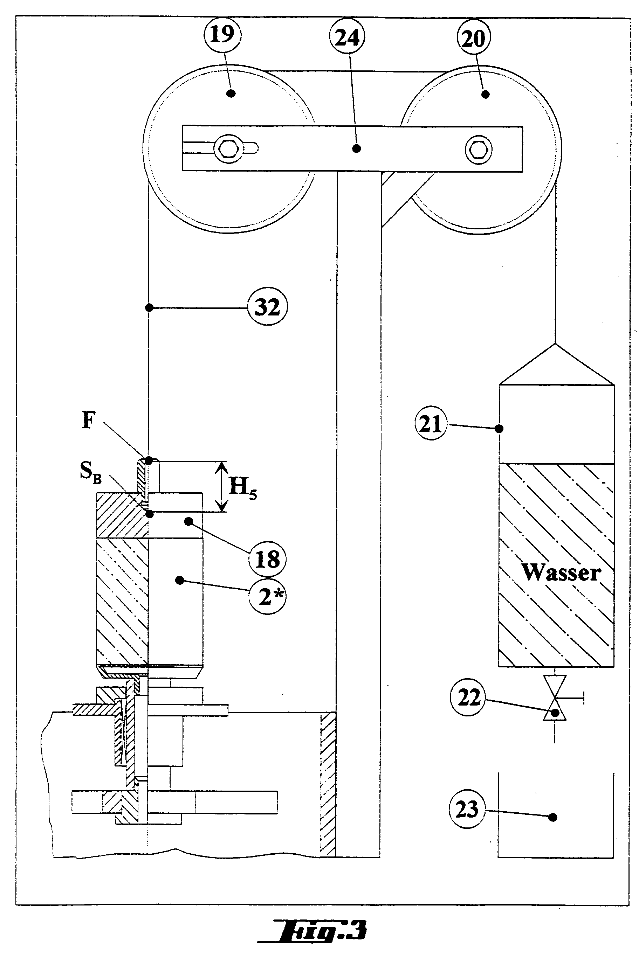

Ein Beispiel für eine einfache Vorrichtung zur Aufbringung der Bruchlast ausgerüstet zeigt Fig. 3.

An einem Kragarm (24) sind zwei kugelgelagerte Umlenkrollen (19, 20) angeordnet, über die eine dünne, biegeschlaffe Schnur (32) ausreichender Reißfestigkeit geführt wird. An deren einem Ende ist die Bruchlast (18), an dem anderen ein aus dünnen Blech gefertigter Wasserbehälter (21) angeordnet.

Zu Meßbeginn sind Bruchblast (18) und Wasserbehälter (21) im Gleichgewicht Nach Aufsetzen der Bruchlast auf Schüttgutprobe (2*) wird das Auslaufventil (22) geöffnet. Das Auslaufventil wird im Moment des Bruches geschlossen, die im Auffanggefäß (23) aufgefangene Wassermenge ausgewogen. Die Druckfestigkeit $f_c$ der Schüttgutprobe (2*) ergibt sich dann aus dem Quotienten der Gewichtskraft der Wassermenge und der Kreisfläche mit Durchmesser D.

Die Genauigkeit, mit der die Druckfestigkeit $f_c$ bestimmt werden kann, hängt vom Durchmesser der Umlenkrollen (19, 20) sowie der Lagerreibung ab. Je größer der Durchmesser der Umlenkrollen, desto empfindlicher ist das System. Bei einem Durchmesser der Umlenkrollen $\geq 150$ mm und Verwendung von Rillenkugellagern ist die Empfindlichkeit besser als $\pm 20$ g. Diese Genauigkeit ist meist ausreichend. Die Umlenkrolle (19) muß konstruktiv so an dem Kragarm (24) angeordnet sein, daß ihre Lage in zwei Horizontalrichtungen einstellbar ist. Dadurch kann die Bruchlast so positioniert werden, daß ihre Achse mit der Achse von Form (1) zusammenfällt. Diese Positionierung muß nur einmal beim Aufstellen der Apparatur vorgenommen werden. Der Aufhängepunkt F der Bruchlast (18) muß wenigstens 40 mm oberhalb des Schwerpunktes der Bruchlast auf der Achse der Bruchlast liegen ($H_5 \geq 40$ mm). Dadurch ist eine horizontale Ausrichtung der Unterseite der Bruchlast (18) gewährleistet. Über den Querschnitt des Auslaufventils (22) kann die Auslaufmenge, d. h. die Beanspruchungsgeschwindigkeit vorgewählt werden.

Zusammenfassend kann nach Beschreibung der Apparatur festgestellt werden, daß im Vergleich mit anderen Geräten zur Bestimmung der Fließfähigkeit von Schüttgüter, insbesondere Translationsschergeräten, Ringschergeräten, Torsionsschergeräten und der Vorrichtung nach WO 94/08220 A1 die erfindungsgemäße Apparatur

- ohne elektrischen Antrieb (hoch untersetzter Getriebemotor o. ä.) auskommt,
- ohne DMS-Meßaufnehmer zur Kraftmessung, Meßverstärker, Schreiber auskommt,
- im wesentlichen aus einer robusten Schweißkonstruktion sowie einfachen Drehteilen besteht, eine Ausnahme ist lediglich die sehr präzise gearbeitete mehrteilige Form (1).

Selbst bei Kleinserienfertigung ist die erfindungsgemäße Apparatur daher vergleichsweise kostengünstig herstellbar und damit für den Einsatz in der produktionsnahen Qualitätskontrolle gut geeignet.

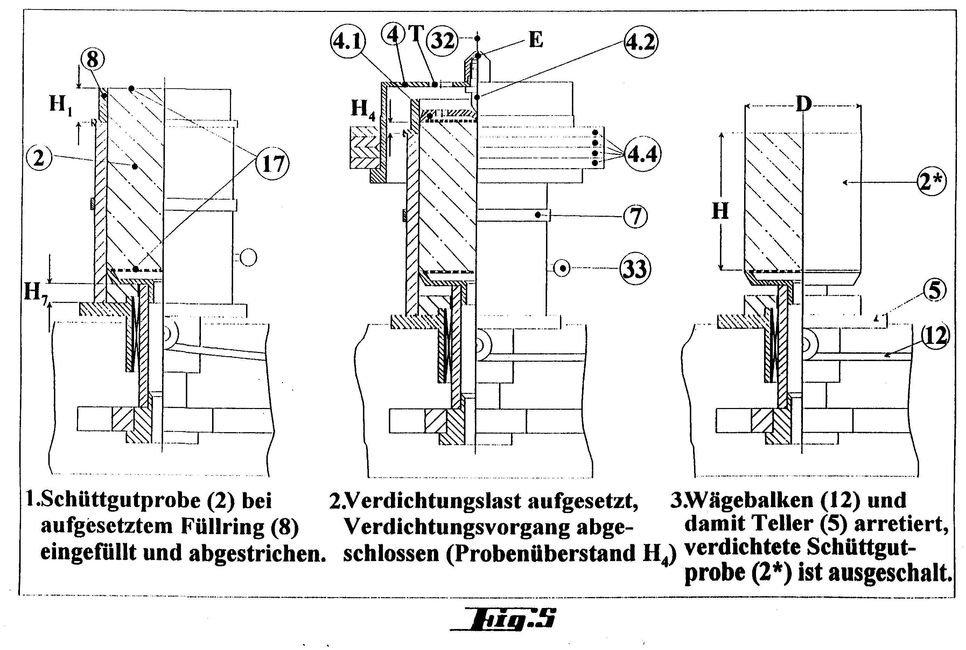

Im folgenden wird kurz die Messung der Fließfähigkeit eines Schüttgutes mit der erfindungsgemäßen Vorrichtung beschrieben (Fig. 5).

- Einfüllen der Schüttgutprobe (2)

Zunächst wird auf Kontergewicht (13) ein Zusatzgewicht (13.1) aufgelegt, welches eine Bewegung des Tellers (5) während des Befüllens der Form (1) verhindert. Teller (5) befindet sich automatisch in seiner obersten Position (Fig. 5, 1). Dann werden die Teile der Form (1) auf den (sauberen) Teller (5) aufgesetzt, an Zentrierring (6) geschoben und durch Spannring (7) fixiert. Als Spannring kann im einfachsten Fall eine Schlauchschelle verwendet werden. Ein Filter (17) mit Durchmesser D wird auf die untere Begrenzungsplatte (15) aufgelegt und der Füllring (8) aufgesetzt. Jetzt wird das Schüttgut eingefüllt.

In manchen Fällen ist ein Durchrühren (von Hand) der Schüttgutprobe unmittelbar vor Einfüllen in die Form (1) sinnvoll. Durch diese kurzzeitig wirksame Verbesserung der Fließfähigkeit (Eintrag von Luft, Reduzierung der zwischen den Partikeln wirksamen Haftkräfte) kann dann eine gleichmäßigere, hohlraumfreie Befüllung der Form (1) erreicht werden. Die Form (1) wird mit leichtem Materialüberschuß befüllt, so daß sich durch Abstreichen des überschüssigen Schüttgutes eine glatte, mit der Oberkante des Füllrings (8) bündige Oberfläche der Schüttgutprobe (2) ergibt. Zum Abstreichen wird ein gerades Messer o. ä. verwendet. Auf die Oberfläche der Schüttgutprobe (2) wird nun ein zweites Filter (17) aufgelegt. Die so für den folgenden Verdichtungsvorgang vorbereitete Probe zeigt Fig. 5, 1.

- Verdichtung der Schüttgutprobe (2)

Auf die Oberfläche der Schüttgutprobe (2) wird vorsichtig der Verdichtungsdeckel (4.1) aufgelegt. Der Verdichtungsdeckel sinkt wegen seines geringen Gewichtes nur leicht ein. Jetzt wird das Zusatzgewicht (13.1) vom Kontergewicht (13) abgenommen.

Nunmehr wird die Verdichtungslast (4) aufgesetzt, wobei die gewünschte Verfestigungsspannung $\sigma_1$ durch Auflegen der entsprechenden Zahl der Zusatzgewichte (4.4) vorgewählt werden kann. Zum Aufsetzen der Verdichtungslast (4) wird die in Fig. 3 gezeigte Vorrichtung zur Aufbringung der Bruchlast genutzt. Die Verdichtungslast (4) ist in Punkt (E) an einer dünnen, reißfesten, biegeschlaffen Schnur (32) aufgehängt. Die Schnur (32) wird über die Umlenkrollen (19, 20) geführt, die Verdichtungslast (4) mittels eines am anderen Ende der Schnur (32) befestigten Handgriffes gehalten. In einer bevorzugten Ausführungsform liegen Aufhängepunkt E, Schwerpunkt S und Kraftangriffspunkt C auf einer lotrechten Geraden.

Die Verdichtungslast (4) wird langsam abgesenkt, wobei über die Öffnungen T der Verdichtungslast (4) kontrolliert werden kann, daß die Spitze des Bolzen (4.2) tatsächlich auf die Mitte des Verdichtungsdeckels aufsetzt. Nach Aufsetzen der Verdichtungslast (4) auf Verdichtungsdeckel (4.1) wird zunächst die obere Schicht der Schüttgutprobe (2) komprimiert, d. h. der Verdichtungsdeckel (4.1) sinkt in den Füllring (8) ein. Anschließend ist zu beobachten, daß sich Form (1) nach unten bewegt, wobei gleichzeitig der Verdichtungsdeckel (4.1) weiter in Füllring (8) einsinkt. Aus Fig. 5, 2 ist unmittelbar verständlich, daß die Schüttgutprobe (2) durch die Relativbewegung zwischen Form (1) und Unterstempel (3) auch von der unteren Stirnfläche her verdichtet wird.

Die Geschwindigkeit, mit der die Verdichtungslast (4) abgesenkt wird, wird anfänglich manuell bestimmt, ist aber ab einem bestimmten Punkt (Seilkraft in Schnur (32) gleich Null) nur noch durch die Luftdurchlässigkeit der verwendeten Filter (17) bestimmt. Durch die Ringspalte zwischen Unterstempel (3) und Form (1) bzw. Verdichtungsdeckel (4.1) und Füllring (8) tritt praktisch kein Schüttgut aus. Die Abwärtsbewegung der Form (1) wird durch Reibungskräfte, die zwischen verdichteten Schüttgutschichten und Form (1) bzw. Füllring (8) wirksam werden, verursacht.

Nach Stillstand der Form (1) wirkt auf die obere Stirnseite der Schüttgutprobe (2) die Verfestigungsspannung $\sigma_1$.

$$\sigma_1 = \frac{\text{Summe Gewichtskräfte Pos. 4, 4.1, 4.2, 4.3, 4.4}}{\text{Kreisfläche mit Durchmesser D}}$$

Auf die untere Stirnseite der Schüttgutprobe wirkt die höhere Verfestigungsspannung $\sigma_{1,U}$.

$$\sigma_{1,U} = \sigma_1 + \frac{\text{Gewichtskraft Schüttgutprobe}}{\text{Kreisfläche mit Durchmesser D}}$$

Da die Gewichtskraft der Form (1) bei der erfindungsgemäßen Vorrichtung über das Kontergewicht (13) gerade ausgeglichen wird, ist die Summe der zwischen Form (1) und Schüttgutprobe (2) wirksamen Wandschubspannung in vertikaler Richtung gerade gleich Null. Trotzdem dürften lokal Wandschubspannungen wirksam sein, die Wandschubspannungsverteilung ist jedoch zunächst nur qualitativ bekannt.

Nach Stillstand von Form (1) ist der Verdichtungsvorgang praktisch abgeschlossen. Zweckmäßigerweise kann eine Verdichtungszeit $t_V$ von z. B. 2 min vorgegeben werden. Die Verdichtungslast (4) kann beliebig lange auf Verdichtungsdeckel (4.1) aufstehen, d. h. mit der erfindungsgemäßen Vorrichtung kann die sog. Zeitverfestigung von Schüttgütern (Erhöhung der Festigkeit der Schüttgutprobe bei Lagerung in Ruhe unter Druck) bestimmt werden. Die Kenntnis des Zeitverfestigungsverhaltens eines Schüttgutes ist insbesondere bei der Auslegung von Silos wichtig.

Fig. 5, 2 zeigt die Schüttgutprobe (2) nach Ablauf der Verdichtungszeit $t_V$.

Jetzt wird Wägebalken (12) mittels Arretierschraube (14) arretiert. Verdichtungslast (4), Verdichtungsdeckel (4.1), Füllring (8) und Filter (17) werden abgenommen. Jetzt wird der Probenüberstand $H_4$ (Fig. 5, 2) gemessen und im Versuchsprotokoll notiert. $H_4$ sollte wenigstens 2 mm, höchstens aber etwa 8 mm betragen, andernfalls ist der Versuch mit einer anderen Füllringhöhe $H_1$ zu wiederholen. Die Füllringhöhe $H_1$ wird ebenfalls im Versuchsprotokoll festgehalten. Für ein bestimmtes Schüttgut, z. B. Kalksteinmehl einer bestimmten Feinheit und Feuchte aus derselben Lagerstätte, muß die geeignete Füllringhöhe normalerweise nur einmal festgestellt werden.

Der Probenüberstand $H_4$ ist über den Umfang nicht gleich, d. h., in praktisch allen Fällen kippt der Verdichtungsdeckel (4.1) während des Verdichtungsvorgangs leicht. Ursache hierfür sind unvermeidbare lokale Unterschiede der Schüttdichte nach dem Einfüllen des Schüttgutes in Form (1). Dort, wo die Schüttgutprobe lockerer gepackt ist und daher stärker komprimiert werden kann, sackt der Verdichtungsdeckel (4.1) stärker ein. Anders ausgedrückt ist die "Tragfähigkeit" der Schüttgutprobe dort schlechter als an den dichter gepackten Stellen der Schüttgutprobe.

Zielsetzung ist eine möglichst gleichmäßige Verdichtung Schüttgutprobe, insofern ist die Möglichkeit des Verdichtungsdeckels (4.1), frei um Kraftangriffspunkt C kippen zu können, zunächst positiv zu beurteilen, da so während des Verdichtungsvorgangs anfänglich vorhandene lokale Unterschiede in der Schüttdichte auf jeden Fall reduziert werden. Andererseits sollte eine "Selbstkorrektur" der Lage des Verdichtungsdeckels (4.1) erfolgen können, d. h. ein anfängliches Kippen des Verdichtungsdeckels (4.1) in eine Richtung sollte nicht zu einer Fixierung dieser Schrägstellung führen. Dies ist jedoch um so eher der Fall, je größer der Abstand $H_2$ des Kraftangriffspunktes C von der Unterseite des Verdichtungsdeckels (4.1) ist. Im Fall der Schrägstellung des Verdichtungsdeckels (4.1) entsteht dann ein Drehmoment bezüglich des Mittelpunktes der Unterseite des Verdichtungsdeckels (4.1), das die Schrägstellung des Verdichtungsdeckels (4.1) zusätzlich unterstützt.

Idealerweise sollte daher der Kraftangriffspunkt C in der Unterseite des Verdichtungsdeckels (4.1) liegen ($H_2 = 0$) oder sogar noch tiefer ($H_2 < 0$). Diese Forderung kann konstruktiv jedoch nicht ohne weiteres erfüllt werden.

Ein nach Fig. 2 ausgeführter Verdichtungsdeckel (4.1) erfüllt mit $H_2 = 3$ mm diese Forderung bereits recht gut. Dementsprechend wurden Differenzen $H_{4, max} - H_{4, min} \leq 6$ mm festgestellt.

Ist der Probenüberstand $H_4$ in Ordnung, so wird er mit einem geraden Messer o. ä. abgestrichen, so daß eine glatte, mit der Oberkante von Form (1) bündige Schüttgutoberfläche entsteht. Anschließend wird Spannring (7) entfernt. Dann werden die Teile der Form (1) nacheinander mittels der Griffe (33) auf Teller (5) radial nach außen gezogen und entfernt. Danach steht die verdichtete, zylindrische Schüttgutprobe (2*) frei auf Unterstempel (3) (Fig. 5, 3).

Bei den bisher durchgeführten Messungen der Fließfähigkeit von Kalksteinmehlen verschiedener Feinheit und Herkunft, verschiedenen Kalkhydraten sowie Weißfeinkalk war in praktisch allen Fällen ein problemlos, beschädigungsfreies Ausschalen der komprimierten Schüttgutprobe (2*) möglich. Dabei war die äußere Oberfläche der komprimierten Schüttgutproben nach dem Ausschalen immer glatt und ohne jegliche sichtbaren Hohlräume o. ä. Damit ist die Eignung der erfindungsgemäßen Vorrichtung zur Messung der Eigenschaften der wesentlichen Produkte der Kalk- und Zementindustrie nachgewiesen.

Jetzt wird die Höhe H der Schüttgutprobe (2*) gemessen und im Versuchsprotokoll vermerkt. Das Verhältnis H/D muß größer als eins sein. Diese Bedingung kann durch geeignete Wahl der Höhe $H_6$ der Form (1) und $H_7$ des Zentrierrings (6) ohne weiteres eingehalten werden.

Für einen exakten Vergleich der Fließfähigkeit verschiedener Schüttgüter ist ein gleiches H/D-Verhältnis erforderlich. Bei vorgegebener Höhe $H_6$ der Form (1) kann diese Forderung durch Verwendung von gewichtsgleichen Zentrierringen (6) verschiedener Höhe $H_7$ erfüllt werden. Der Zentrierring (6) ist einfach herzustellen, liegt nur auf Teller (5) auf und kann durch Abhängen von Kontergewicht (13) und Abschrauben von Unterstempel (3) leicht ausgetauscht werden.

Die zylindrische, frei auf Unterstempel (3) aufstehende Schüttgutprobe (2*), kann durch eine auf die obere Stirnfläche der Schüttgutprobe (2*) wirkende Druckspannung belastet werden, wobei diese Druckspannung langsam und kontinuierlich von Null beginnend gesteigert werden kann und die im Moment des Bruches von Schüttgutprobe (2*) herrschende Druckspannung $f_c$ registriert werden kann. Dabei können aus der Materialprüfung bekannte Konstruktionen verwendet werden. Die mit der erfindungsgemäßen Apparatur meßbaren Druckspannungen $f_c$ können jedoch vergleichsweise niedrig liegen (unter 500 Pa), was eine entsprechende Empfindlichkeit der Druckmessung erfordert.

Wie in Fig. 3 dargestellt wird die Wassermasse in Wasserbehälter (21) über das Auslaufventil (22) so eingestellt, daß die Gewichtskräfte von Bruchlast (18) und Wasserbehälter (21) gleich sind, d. h. Bruchlast (18) in Ruhe ist. Dann wird Bruchlast (18) vorsichtig auf die Oberfläche von Schüttgutprobe (2*) geführt.

Dann wird Auslaufventil (22) geöffnet, und das auslaufende Wasser in dem zuvor ausgewogenen Auffanggefäß (23) aufgefangen. Die Gewichtskraft der in Auffanggefäß (23) aufgefangenen Wassermasse $m_{H2O}$ entspricht der auf die Oberseite von Schüttgutprobe (2*) wirkenden Kraft.

Die Auslaufgeschwindigkeit und damit die Beanspruchungsgeschwindigkeit nimmt mit Abnahme der Wassermenge in Wasserbehälter (21) ab. Das ist erwünscht. Darüber hinaus kann die Beanspruchungsgeschwindigkeit durch den Querschnitt von Auslaufventil (22) beeinflußt werden. Im Moment des Bruches von Schüttgutprobe (2*) - der Bruch erfolgte in allen bisher durchgeführten Versuchen schlagartig - wird Auslaufventil (22) geschlossen, die in Auffanggefäß (23) aufgefangene Wassermasse $m_{H2O,B}$ ausgewogen. Die Druckfestigkeit $f_C$ der Schüttgutprobe ergibt sich aus

$$f_C = \frac{g \cdot m_{H2O,B}}{\frac{\pi}{4} D^2} \text{ mit } g = \text{Erdbeschleunigung}$$

Die Fließfähigkeit $ff_C$ des Schüttgutes ergibt sich entsprechend der Definition nach JENIKE aus

$$ff_C = \frac{\sigma_1}{f_C}$$

oder direkt aus

$$ff_C = \frac{\text{Masse Verdichtungslast (Pos. 4 + 4.1 + 4.2 + 4.3 + 4.4)}}{m_{H2O,B}}$$

In manchen Fällen kann die Masse der komprimierten Schüttgutprobe (2*) von Interesse sein. Da die Abmessungen der Schüttgutprobe (2*) (Durchmesser D, Höhe H) und damit deren Volumen bekannt ist, kann dann die Schüttdichte der Probe (2*) als Funktion der Verfestigungsspannung $\sigma_1$ nach

$$\rho_s(\sigma_1) = \frac{m_{2*}}{V_{2*}}$$

angegeben werden.

Soll die Schüttdichte $\rho_s(\sigma_1)$ bestimmt werden, so werden vor dem Ausschalen der Behälter (35), (die Masse von Behälter (35) ist durch Wägung bekannt) Rohrstück (30) und Trichter (34) gesäubert. Nach Zerstörung der Schüttgutprobe (2*) wird diese in Behälter (35) aufgefangen, wobei anhaftendes Schüttgut von Teller (5), Rohrstück (30), Trichter (34) usw. sorgfältig abgepinselt wird. Masse $m_{2*}$ wird durch Wägung bestimmt, so daß $\rho_s(\sigma_1)$ berechnet werden kann.

Nun kann auch die manchmal verwendete Kenngröße

$$\frac{f_C(\sigma_1)}{g \cdot \rho_s(\sigma_1)}$$

bestimmt werden.

Tabelle 1 zeigt das Ergebnis von Fließfähigkeitsmessungen für verschiedene Kalksteinmehle (KStM) mit der erfindungsgemäßen Apparatur.

Die Steinmehle A1 und A2 stammen aus derselben Lagerstätte und unterscheiden sich lediglich in der Körnung (Feuchte gleich). Wegen der deutlich höheren Feinheit ist für KStM A1 eine niedrigere Fließfähigkeit, d. h. ein kleinerer $ff_C$-Wert, als für KStM A2 zu erwarten (Haftkräfte zwischen Partikel wachsen mit abnehmender Partikelgröße). Diese physikalische Erwartung, die im übrigen praktischer Erfahrung entspricht, wird durch die gemessenen $ff_C$-Werte bestätigt.

Bei der Messung der Fließfähigkeit nach IMSE muß zunächst eine geeignete Siebmaschenweite gefunden werden. Bei Siebmaschenweite 200 µm findet man nach IMSE für KStM A1 eine höhere Fließfähigkeit als für KStM A2, dies Ergebnis widerspricht der praktischen Erfahrung. Bei höheren Siebmaschenweiten (315, 500 µm) findet man die umgekehrten Verhältnisse, jedoch sind die Unterschiede außerordentlich gering, d. h. angesichts der mit der IMSE-Methode erreichbaren Genauigkeit beurteilt das IMSE-Verfahren die Fließfähigkeiten der Steinmehle A1 und A2 gleich. Hier zeigt sich die mangelnde Differenzierungsfähigkeit der IMSE-Methode. Es wird deutlich, daß Vergleichsmessungen für Schüttgüter unterschiedlicher Feinheit nach der IMSE-Methode wenig sinnvoll sind.

Hingegen ist für den Fachmann klar, daß KStM A1 mit einer Fließfähigkeit $ff_C$ = 3,48 sich schüttguttechnisch deutlich von KStM A2 mit $ff_C$ = 5,39 unterscheidet.

Die erfindungsgemäße Apparatur ermöglicht also gegenüber der IMSE-Methode eine wesentlich bessere Differenzierung der Fließfähigkeit. Dies wird bestätigt, wenn man KStM B, das aus einer anderen Lagerstätte stammt, in der Betrachtung einbezieht. KStM B liegt im Oberkorn ($d_{97}$) ähnlich wie KStM A2, enthält aber gegenüber KStM A2 mehr Feinstgut. Der Feinstgutgehalt kann beispielsweise durch den Durchgangssummenwert der Korngrößenverteilung bei x = 10 µm, $Q_3$ (10 µm), charakterisiert werden.

Aufgrund der Korngrößenverteilung ist für KStM B eine Fließfähigkeit zwischen KStM A1 und A2 zu erwarten. Die gemessenen $ff_C$-Werte entsprechen der Erwartung, die Fließfähigkeit nimmt mit zunehmendem Feinstanteil $Q_3$ (10 µm) ab. Ein solch physikalisch begründeter Zusammenhang kann mit der IMSE-Methode nicht gefunden werden. Insbesondere müßte nach IMSE KStM A1 eine höhere Fließfähigkeit als KStM B aufweisen, was praktischer Erfahrung widerspricht.

Die Messung für KStM A1 wurde fünfmal wiederholt, in Tab. 1 ist der Mittelwert angegeben. Die maximale Abweichung eines Einzelwertes vom Mittelwert betrug ± 2,5 %. Diese Reproduzierbarkeit bei der Messung einer summarischen Schüttguteigenschaft wie der Fließfähigkeit ist als gut zu beurteilen.

Hervorzuheben ist nochmals, daß mit der erfindungsgemäßen Apparatur für verschiedene Schüttgüter gemessene Fließfähigkeiten direkt verglichen werden können. Dabei ist lediglich zu beachten, daß die Verfestigungsspannung $\sigma_1$ gleich ist und für exakte Vergleiche das H/D-Verhältnis gleich sein sollte.

Tabelle 1
Fließfähigkeit verschiedener Kalksteinmehle

| Schüttgut | $\sigma_1$ N/m² | $f_c$ N/m² | $ff_c$ - | H/D - | $d_{97}$ μm | $d_{50}$ μm | $Q_3$ (10 μm) % | Feuchte % | FF nach IMSE in % | | |
|---|---|---|---|---|---|---|---|---|---|---|---|
| | | | | | | | | | 200 μm | 315 μm | 500 μm |
| KStM A1 | 3935 | 1131 | 3,48 | 1,16 | 41,8 | 11,7 | 43,1 | 0,07 | 13 | 28 | 70 |
| KStM B | 3935 | 865 | 4,55 | 1,16 | 128,2 | 15,67 | 37,34 | 0,12 | 7,5 | 25 | 64 |
| KStM A2 | 3935 | 730 | 5,39 | 1,19 | 135,7 | 21,7 | 27,04 | 0,07 | 11,5 | 29 | 72 |

$\sigma_1$ = Verfestigungsspannung  
$f_c$ = Druckfestigkeit der zylindrischen, freistehenden Probe (2*)

$$ff_c = \frac{\sigma_1}{f_c}$$

D = Durchmesser Schüttgutprobe (= 95 mm)  
H = Höhe Schüttgutprobe (2*)

Verdichtungszeit : 2 min  
Korngrößenbest. : Beugungsspektrometer HELOS (Fa. Sympatec)

$d_{97}$ : Oberkorn ($Q_3(d_{97})$ = 97 %)  
$d_{50}$ : mittlere Korngröße  
$Q_3$(10 μm) : Massenanteil < 10 μm (Durchgangssumme bei x = 10 μm)

Fließfähigkeit nach IMSE in % = Siebdurchgang in %

Bezugszeichenliste

(1)        Form

10

| | |
|---|---|
| (2) | Schüttgutprobe |
| (2*) | freistehende Schüttgutprobe |
| (3) | Unterstempel |
| (4) | Verdichtungslast |
| (4.1) | Verdichtungsdeckel |
| (4.2) | Bolzen |
| (4.3) | Kappe |
| (4.4) | Zusatzgewichte |
| (5) | Teller |
| (6) | Zentrierring |
| (7) | Spannring |
| (8) | Füllring |
| (9) | Linearkugellager |
| (10) | Hohlwelle |
| (11) | Kugellager |
| (12) | Wägebalken |
| (13) | Kontergewicht |
| (13.1) | Zusatzgewicht |
| (14) | Arretierschraube |
| (15) | untere Begrenzungsplatte |
| (16) | obere Begrenzungsplatte |
| (17) | Filter |
| (18) | Bruchlast |
| (19, 20) | Umlenkrollen |
| (21) | Wasserbehälter |
| (22) | Auslaufventil Wasserbehälter (21) |
| (23) | Auffanggefäß |
| (24) | Kragarm |
| (25) | Stellfüße |
| (30) | Rohrstück |
| (31) | Haltering |
| (32) | Schnur |
| (33) | Griff |
| (34) | Trichter |
| (35) | Behälter |
| A | Drehpunkt Wägebalken (12) |
| B | Kraftangriffspunkt des Kontergewichtes (13) an Wägebalken (12) |
| C | Kraftangriffspunkt von Verdichtungslast (4) an Verdichtungsdeckel (4.1) |
| D | Innendurchmesser Form (1), Durchmesser Schüttgutprobe (2, 2*) |
| $D_1$ | Innendurchmesser Füllring (8) |
| $D_2$ | Außendurchmesser Verdichtungsdeckel (4.1) |
| E | Aufhängepunkt von Verdichtungslast (4) an Verdichtungsdeckel (4.1) |
| F | Aufhängungspunkt Bruchlast (18) |
| K | Kanal |
| R | Bohrungen Verdichtungsdeckel (4.1) |
| $S_B$ | Schwerpunkt Verdichtungsdeckel |
| $S_V$ | Schwerpunkt Bruchlast |
| T | Öffnungen Verdichtungslast (4) |
| H | Höhe Schüttgutprobe (2*) |
| $H_1$ | Höhe Füllring (8) |
| $H_2$ | Abstand Punkt C zur Unterseite Verdichtungsdeckel (4.1) |
| $H_3$ | Abstand Punkt $S_V$ zu Punkt C |
| $H_4$ | Probenüberstand |
| $H_5$ | Abstand Punkt $S_B$ zu Punkt F |
| $H_6$ | Höhe Form (1) |
| $H_7$ | Höhe Zentrierring (6) |

**Patentansprüche**

1. Vorrichtung zur Messung der Eigenschaften von Schüttgütern nach dem Prinzip des einachsigen vertikalen Druckversuchs, die einen Unterstempel, eine hohlzylindrische Form (1) und einen Verdichtungsdeckel (4.1) aufweist, wobei die hohlzylindrische Form (1) nach dem Verdichten ohne Beschädigung der Schüttgutprobe (2) entfernt werden kann, dadurch gekennzeichnet, daß die Form (1) gegenüber dem feststehenden Unterstempel (3) während des Verdichtungsvorganges in Belastungsrichtung verschiebbar ist.

2. Vorrichtung nach Anspruch 1, dadurch gekennzeichnet, daß die Gewichtskraft der in Belastungsrichtung gegenüber dem Unterstempel (3) verschiebbaren Form (1) in jeder Position durch eine gleich große Gegenkraft genau kompensierbar ist.

3. Vorrichtung nach Anspruch 1 oder 2, dadurch gekennzeichnet, daß die Form (1) mehrteilig, vorzugsweise mindestens dreiteilig ist, die Teilfugen parallel zur Zylinderachse verlaufen und in einem Schnitt senkrecht zur Zylinderachse mit Radialstrahlen zusammenfallen.

4. Vorrichtung nach einem der Ansprüche 1 bis 3, dadurch gekennzeichnet, daß die Innenfläche der Form (1) mit einer Edelstahlfolie ausgekleidet ist.

5. Vorrichtung nach einem der Ansprüche 1 bis 4, dadurch gekennzeichnet, daß die beiden stirnseitigen Begrenzungsplatten (15, 16) der Schüttprobe (2) luftdurchlässig sind.

6. Vorrichtung nach einem der Ansprüche 1 bis 5, dadurch gekennzeichnet, daß zwischen den durchbrochenen Begrenzungsplatten (15, 16) und den Stirnseiten der Schüttgutprobe (2) ein leicht auswechselbares Filter (17) angeordnet ist.

7. Vorrichtung nach Anspruch 6, dadurch gekennzeichnet, daß als leicht auswechselbares Filter ein Filtergewebe, Filtervlies oder Filterpapier eingesetzt ist.

**Fig. 1**

Erdung

Schnitt X-X

**Fig.2**

Fig.3

Schnitt Z-Z

Schnitt Y-Y

Fig. 4

1. Schüttgutprobe (2) bei aufgesetztem Füllring (8) eingefüllt und abgestrichen.

2. Verdichtungslast aufgesetzt, Verdichtungsvorgang abgeschlossen (Probenüberstand $H_4$)

3. Wägebalken (12) und damit Teller (5) arretiert, verdichtete Schüttgutprobe (2*) ist ausgeschalt.

Fig.5

EP 0 763 726 A2